Europäisches Patentamt

(19) European Patent Office

Office européen des brevets

(11) Publication number: **0 101 562**
A2

(12) # EUROPEAN PATENT APPLICATION

(21) Application number: 83107024.8

(22) Date of filing: 18.07.83

(51) Int. Cl.³: **A 61 K 39/02,** A 61 K 39/05,
A 61 K 39/08, A 61 K 39/10,
A 61 K 39/102

(30) Priority: 20.08.82 US 409776

(71) Applicant: **AMERICAN CYANAMID COMPANY,**
**1937 West Main Street P.O. Box 60, Stamford**
**Connecticut 06904 (US)**

(43) Date of publication of application: **29.02.84**
**Bulletin 84/9**

(72) Inventor: **Kuo, Joseph S.C., 104 Rutgers Road,**
**Orangeburg New York (US)**

(84) Designated Contracting States: **BE CH DE FR GB IT LI**
**NL SE**

(74) Representative: **Wächtershäuser, Günter, Dr., Tal 29,**
**D-8000 München 2 (DE)**

(54) **Combined haemophilus influenzae and diphtheria, pertussis, tetanus vaccine.**

(57) A vaccine for eliciting polyribosyl ribitol phosphate
(PRP) antibody formation in a warm-blooded animal has
been invented. The vaccine comprises the capsular polysaccharide PRP isolated and purified from Haemophilus
influenzae type b combined with a diphtheria, Bordetella
pertussis and tetanus (DPT) vaccine.

A method of making a vaccine for eliciting polyribosylribitol phosphate (PRP) antibody formation in a warm-
blooded animal has also been invented. The method of
making comprises obtaining purified PRP; dissolving the
PRP in phosphate buffered saline solution having a pH of
about 7.0; sterile filtering the PRP solution; and combining
the PRP solution with a diphtheria, B. pertussis and tetanus
(DPT) vaccine.

Finally, a method for inducing active immunization in
a warm-blooded animal against systemic infection caused
by the pathogen H. influenzae type b has been invented. The
method of using comprises administering an immunogenic
amount of a vaccine comprising the capsular polysaccharide polyribosyl ribitol phosphate (PRP) isolated and
purified from H. influenzae type b combined with a diphtheria, B. pertussis and tetanus (DPT) vaccine.

## COMBINED HAEMOPHILUS INFLUENZAE AND
## DIPHTHERIA, PERTUSSIS, TETANUS VACCINE
## BACKGROUND AND SUMMARY OF THE INVENTION

Haemophilus influenzae type b is the most common cause of bacterial meningitis. It is also responsible for an array of bacteremic and sometimes life-threatening infections of young children.

H. influenzae type b caused meningitis is predominantly a disease of children less than 5 years old. The highest incidence is in infancy, e.g. in 6 to 18 month old infants.

A vaccine for eliciting polyribosyl ribitol phosphate (PRP) antibody formation in a warm-blooded animal has been invented. The vaccine comprises the capsular polysaccharide PRP isolated and purified from Haemophilus influenzae type b combined with a diphtheria, Bordetella pertussis and tetanus (DPT) vaccine. In other embodiments, the PRP is obtained from H. influenzae type b CK strain or from H. influenzae type b Rab strain, and the DPT vaccine is Tri-Immunol®. In another embodiment, the warm-blooded animal is a human child.

A method of making a vaccine for eliciting polyribosylribitol phosphate (PRP) antibody formation in a warm-blooded animal has also been invented. The method of making comprises obtaining purified PRP; dissolving the PRP in a phosphate buffered saline solution having a pH of about 7.0; sterile filtering the PRP solution; and combining the PRP solution with a diphtheria, B. pertussis and tetanus (DPT) vaccine. In one embodiment, the method

- 2 -

0101562

comprises after the obtaining step, lyophilizing the PRP. In other embodiments, the PRP is obtained from H. influenzae type b CK strain or from H. influenzae type b Rab strain, and the DPT vaccine is Tri-Immunol®. In another embodiment, the warm-blooded animal is a human child.

Finally, a method for inducing active immunization in a warm-blooded animal against systemic infection caused by the pathogen H. influenzae type b has been invented. The method of using comprises administering an immunogenic amount of a vaccine comprising the capsular polysaccharide polyribosyl ribitol phosphate (PRP) isolated and purified from H. influenzae type b combined with a diphtheria, B. pertussis and tetanus (DPT) vaccine. In other embodiments, the warm-blooded animal is a human child and the infection is meningitis.

DETAILED DESCRIPTION

The PRP used in the vaccine is prepared by methods known in the prior art.

The DPT vaccine which is combined with the PRP is commercially available, e.g. Tri-Immunol®, American Cyanamid Company, Wayne, New Jersey, U.S.A.

In the examples, the combined vaccine was prepared at a temperature of 4°C. However, it is to be understood that other temperatures can be used to prepare the combined vaccine, e.g. 5°C or ambient temperature.

Also in the examples, sucrose is added to a PRP stock solution prior to lyophilization. The sucrose is added as a body material. However, it is to be understood that the sucrose may have other uses, e.g. as a stabilizer for the PRP stock solution. It is also to be understood that other compositions can be added to a PRP stock solution as a body material. Examples of other compositions are glycine and glycine plus sucrose. The amount of composition added to the PRP stock solution is about the same as the amount of sucrose added. When the composition is a mixture, e.g. glycine and sucrose, the amount of each compound added is approximately equal.

## Example 1

### Preparation of PRP + DPT Vaccine

A stock solution of PRP (100 μg PRP/ml) was prepared by dissolving 20 mg of PRP in 200 ml of sterile phosphate buffered saline (PBS) and filtering through 0.45 μ and 0.22 μ millipore filters. A commercially availabe DPT vaccine (Tri-Immunol®) containing approximately 25 Lf of diphtheria toxoid, 10 Lf of tetanus toxoid, 24 opacity units of pertussis and 0.8 mg of aluminum phosphate per ml was used. [Lf is a quantitative unit of antigen potency and is that amount of antigen that gives optimal flocculation when reacted with one unit of standard antitoxin. One opacity unit is equivalent to $1 \times 10^9$ pertussis cells.]

The combined vaccine was prepared by mixing 140 ml of PRP stock solution with 700 ml of DPT vaccine at 4°C. The final combined vaccine consisted of 10 μg PRP, 12 opacity units of pertussis, 5 Lf of tetanus toxoid and 12.5 Lf of diphtheria toxoid per 0.6 ml dose.

## Example 2

### Serum Antibody to PRP in Young Rats

### Injected with PRP, PRP + Pertussis and

### PRP + DPT Vaccines

Five Sprague-Dawley rats (4 weeks old) per group were injected with dose per week for three weeks of the vaccine described in Example 1. Three and four weeks after the initial injection, the serum antibody to PRP was determined by radioimmunoassay. The results of the antibody response for each vaccine are described in Table 1.

0101562

- 4 -

TABLE 1

| Vaccine | Antibody to PRP ($\mu$g/ml) Average of 5 Results | |
| | Weeks Post Initial Injection | |
| | 3 | 4 |
|---|---|---|
| PRP + DPT | 840 | 646 |
| PRP + pertussis[1] | 778 | 593 |
| PRP[2] | 212 | 89 |

[1] The vaccine contained 10 g of PRP and from 3.5 to 4.0 opacity units of pertussis, per 0.5 ml dose.

[2] The vaccine had a concentration of 10 g of PRP per 0.5 ml dose.

### Example 3

<u>Serum Antibody to PRP in Children Less Than One year Old Injected with DPT, PRP + Pertussis and PRP + DPT Vaccines</u>

Children were injected with 0.6 ml of the vaccines described in Example 2 as follows: one dose at 2 months of age, one dose at 4 months of age and a final dose at 6 months of age. Blood samples from each child were then taken at 7 months of age. The serum antibody to PRP was determined by radioimmunoassay as described in Example 2. The results of the antibody response for each vaccine are described in Table 2.

0101562

## Table 2

| Vaccine | Child No. | Antibody to PRP for Individual Child (ng/ml) 1 Month Post Third Injection |
|---------|-----------|------------------------|
| PRP + DPT | 1 | 10720 |
| " | 2 | 7200 |
| " | 3 | 3560 |
| " | 4 | 3440 |
| " | 5 | 2880 |
| " | 6 | 1880 |
| " | 7 | 1600 |
| " | 8 | 740 |
| " | 9 & 10 | 700 |
| " | 11 | 390 |
| " | 12 & 13 | 340 |
| " | 14 & 15 | 280 |
| " | 16 | 270 |
| " | 17 | 230 |
| " | 18 | 190 |
| " | 19 | 180 |
| " | 20 & 21 | 160 |
| " | 22 | 150 |
| " | 23 | 140 |
| " | 24 | 110 |
| " | 25 & 26 | 90 |
| PRP + pertussis | 27 | 580 |
| " | 28 | 200 |
| " | 29 | 150 |
| " | 30 | 130 |
| " | 31 | 90 |
| " | 32 & 33 | 80 |
| " | 34 & 35 | 70 |
| " | 36 | 50 |
| " | 37 | 40 |
| " | 38 to 43 | <40 |
| " | 44 | 70 |
| " | 45 to 53 | <40 |

## Example 4
### Preparation and Reconstitution of Lyophilized PRP Stock
### Solution

A PRP stock solution was prepared at a concentration of 40 µg/ml. The PRP was prepared as described in U.S. Patent 4,220,717. To this stock solution was added 1600 µg of sucrose. The stock solution was then dispensed in 1.5 ml portions into 5 ml vials and lyophilized by conventional procedures. Each vial contained 60 µg of PRP.

## Example 5
### Reconstitution of Lyophilized
### PRP Stock Solution with DPT Vaccine

Fifteen vials of the lyophilized PRP stock solution described in Example 4 were each reconstituted with 3 ml of DPT vaccine (Tri-Immunol®). The final PRP-DPT vaccine consisted of 10 µg of PRP and 12 opacity units of pertussis per 0.5 ml dose.

## Example 6
### Serum Antibody to PRP in Young Rats Injected with
### PRP, Reconstituted PRP + Pertussis and Reconstituted
### PRP + DPT Vaccines

Five Sprague-Dawley rats (4 weeks old) per group were injected with 1 dose per week for three weeks of the vaccine described in Example 4. Three and four weeks after the initial injection, the serum antibody to PRP was determined by radioimmunoassay as described in J. Immunol. Methods 43 33-47 (1981). The results of the antibody to PRP ratio for each vaccine are described in Table 3.

Table 3

| | Antibody to PRP (ng/ml) Average of 5 Results | |
| | Weeks Post Initial Injection | |
| Vaccine | 3 | 4 |
|---|---|---|
| PRP + DPT | 624 | 486 |
| PRP + pertussis[1] | 520 | 363 |
| PRP[2] | 122 | 132 |

[1]Obtained by reconstituting the lyophilized PRP of Example 4 with 3 ml. of B. pertussis per vial. The combined vaccine had 10 μg of PRP and 4 opacity units of pertussis per 0.5 ml dose.

[2]Obtained by reconstituting the lyophilized PRP of Example 4 with 3 ml. of phosphate buffered saline (PBS) per vial.

0101562

- 8 -

WE CLAIM

1. A vaccine for eliciting polyribosyl ribitol phosphate (PRP) antibody formation in a warm-blooded animal comprising the capsular polysaccharide PRP isolated and purified from Haemophilus influenzae type b combined with a diphtheria, Bordetella pertussis and tetanus (DPT) vaccine.

2. A vaccine of Claim 1 wherein said PRP is obtained from H. influenzae type b CK strain.

3. A vaccine of Claim 1 wherein said PRP is obtained from H. influenzae type b Rab strain.

4. A vaccine of Claim 1 or 2 or 3 wherein said DPT vaccine is Tri-Immunol®.

5. A method of making a vaccine for eliciting polyribosylribitol phosphate (PRP) antibody formation in a warm-blooded animal comprising: .

obtaining purified PRP;

dissolving said PRP in a phosphate buffered saline solution having a pH of about 7.0;

sterile filtering the PRP solution; and combining said PRP solution with a diphtheria, B. pertussis and tetanus (DPT) vaccine.

6. A method of Claim 5 comprising after the obtaining step, lyophilizing said PRP.

7. A method of Claim 5 or 6 wherein said PRP is obtained from the group consisting of H. influenzae type b CK strain or H. influenzae type b Rab strain.

8. A method of Claim 5 or 6 or 7 wherein said DPT vaccine is Tri-Immunol®.

9. A method for inducing active immunization in a warm-blooded animal against systemic infection caused by the pathogen H. influenzae type b comprising administering an immunogenic amount of a vaccine comprising the capsular polysaccharide polyribosyl ribitol phosphate (PRP) isolated and purified from H. influenzae type b combined with a diphtheria, B. pertussis and tetanus (DPT) vaccine.

0101562

- 9 -

10.  A method of Claim 9 wherein said warm-blooded animal is a human child.